(19) Europäisches Patentamt
European Patent Office
Office européen des brevets

(11) EP 3 651 722 B1

(12) # EUROPEAN PATENT SPECIFICATION

(45) Date of publication and mention of the grant of the patent:
**02.02.2022 Bulletin 2022/05**

(21) Application number: **18727297.6**

(22) Date of filing: **28.05.2018**

(51) International Patent Classification (IPC):
***A61K 8/02*** *(2006.01)* ***A61K 8/19*** *(2006.01)*
***A61K 8/26*** *(2006.01)* ***A61K 8/49*** *(2006.01)*
***A61Q 11/00*** *(2006.01)*

(52) Cooperative Patent Classification (CPC):
**A61K 8/0266; A61K 8/19; A61K 8/494; A61Q 11/00;** A61K 2800/412; A61K 2800/43; A61K 2800/58; A61K 2800/612

(86) International application number:
**PCT/EP2018/063887**

(87) International publication number:
**WO 2019/011512 (17.01.2019 Gazette 2019/03)**

(54) **A COMPOSITE MATERIAL FOR WHITENING TEETH AND COMPOSITION COMPRISING THE SAME**

VERBUNDWERKSTOFF ZUM AUFHELLEN VON ZÄHNEN UND ZUSAMMENSETZUNG DAMIT

MATÉRIAU COMPOSITE POUR LE BLANCHIMENT DES DENTS ET COMPOSITION LE COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.07.2017 PCT/CN2017/092754**
**02.08.2017 EP 17184567**

(43) Date of publication of application:
**20.05.2020 Bulletin 2020/21**

(73) Proprietors:
- **Unilever Global IP Limited**
**Wirral, CH62 AZD (GB)**
Designated Contracting States:
**CY DE GB IE IT MT**
- **Unilever IP Holdings B.V.**
**3013 AL Rotterdam (NL)**
Designated Contracting States:
**AL AT BE BG CH CZ DK EE ES FI FR GR HR HU IS LI LT LU LV MC MK NL NO PL PT RO RS SE SI SK SM TR**

(72) Inventors:
- **GHOSH DASTIDAR, Sudipta**
**Bangalore 560 066 (IN)**
- **HU, Qinghong**
**Shanghai**
**200335 (CN)**
- **KUMAR, Praveen**
**Bangalore 560 066 (IN)**
- **PALANISAMY, Bharath**
**Bangalore 560 066 (IN)**
- **ROY, Arindam**
**Bangalore 560 066 (IN)**

(74) Representative: **Tansley, Sally Elizabeth et al**
**Unilever N.V.**
**Unilever Patent Group**
**Bronland 14**
**6708 WH Wageningen (NL)**

(56) References cited:
**WO-A1-2011/036029**
**WO-A2-2012/123241**
**CN-A- 106 629 867**
**US-A- 5 645 629**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 651 722 B1

## Description

### Field of the Invention

The present invention relates to a material and a composition for whitening teeth.

### Background of the Invention

The colour of our teeth is influenced by a combination of their intrinsic colour and the presence of any extrinsic stains that may form thereon. Staining is linked to the adsorption of materials into the pellicle on the surface of the enamel. Factors that influence extrinsic stains include improper brushing techniques, smoking, dietary intake of coloured foods (e.g. red wine), age and the use of certain cationic agents such as chlorhexidine or salts of tin and iron. Consumers have strong desire for whiter teeth and some individuals are unhappy about the inherent colour of their teeth or with the stains. This desire for whiter teeth has led to a growing trend of whitening products which range from toothpastes to mouthwashes and chewing gums.

Toothpastes meant for whitening the teeth rely on an optimization of abrasive and chemical components for the removal of stains. During brushing these abrasive particles get temporarily trapped between the toothbrush and the stained surface (of teeth) to abrade the stains. Chemical components may also be used, usually in conjunction with abrasive particles, and these include calcium chelators, polymers, surfactants, enzymes and oxidising agents.

The pigment Blue Covarine (a phthalocyanine blue pigment) is used in the toothpaste named White Now® and there have been attempts to enhance the deposition of this pigment on teeth. WO12/123241 A1 discloses the use of poly-sulfonic acid for this purpose.

EP1935395 B1 (Unilever) discloses a novel optical approach to tooth whitening. Upon brushing with the toothpaste described in this publication, a blue pigment (in particular blue covarine) is deposited on the teeth surface, where alters the optical effects and enhances the perception of whiteness. This toothpaste is intended to produce temporary tooth whitening effect that can be reapplied as frequently as desired, as it does not contain harsh chemicals, but is not intended to produce permanent changes to the colour of the teeth. Deposition of the blue pigment on the teeth is enhanced by the use of a deposition aid which is most preferably a GANTREZ® type polymer, i.e. a co-polymer of maleic anhydride with methyl vinyl ether.

In some teeth whitening compositions, a dye is used instead of pigments.

US2013129642 A (Unilever) discloses an oral care composition suitable for temporary whitening effect. The composition comprises a continuous phase comprising water or polyhydric alcohol or a mixture thereof; a whitening agent which is dispersed in the continuous phase, and a deposition aid for the whitening agent. The tooth surface whitening agent is a lake dye formed by fixing a dye on a particulate inorganic substrate. The dye is a triarylmethane having blue to green-blue colour with hue angle ranging from 180 to 270 degrees. The amount of lake dye is at least 0.015 wt% of the total weight of the composition.

US20160331653 A1 (Colgate-Palmolive) discloses a tooth whitening oral are composition comprising a triarylmethane dye and core-shell silica particles. Silica forms the core which is etched with a metal silicate. When the surface chemistry of silica is modified with a strong base, the particle increases the retention of blue dye on the surface of teeth, thereby providing a novel whitening agent.

US20161296434 A1 discloses complex of a triarylmethane dye in with a magnesium alkali metal silicate clay for use in oral care products. The water soluble dye is complexed with the insoluble particulate material which leads to whitening effects in oral care products. In particular, the invention utilizes magnesium alkali metal silicate clay, for example a hectorite clay, e.g., laponite, which exhibits a dye absorption and retention compared to others.

WO2012/123241 A2 provides an oral care composition suitable for delivering a temporary whitening effect to the surface of teeth, the composition comprising: a continuous phase comprising water or polyhydric alcohol or a mixture thereof; a particulate tooth surface whitening agent which is dispersed in the continuous phase, and a deposition aid for the particulate tooth surface whitening agent; characterised in that the deposition aid is a poly-(sulphonic acid) polymer having: - [CH2-C(R)(C(O)NH-C(R1)(R2)-CH2-SO3X)]- monomers in its structure, where R is hydrogen or methyl; X is selected from hydrogen, ammonium, alkali metals or alkaline earth metals; and R1 and R2 are each independently selected from hydrogen and alkyl groups of 1 to 4 carbon atoms; and in which the particulate tooth surface whitening agent is a phthalocyanine blue pigment.

WO11036029 A1 relates to a particulate fluorescer and a process for preparing the same, more particularly to a bipolar particulate fluorescer for use in laundry detergent composition, fabric conditioner and cosmetic compositions and a process for making the same.

Luo et al. proposed a modified version of the CIE whiteness index in Garcia JA, Rubino M, Romero J, Hita E. Measuring The Whiteness Of Human Teeth. Color Research and Application 1993; 18: 349 - 52. It is based directly on observers scoring of the Vita® 3D Shade Guide and is defined as:

$$WIO = Y + 1075.012 (x_p - x) + 145.516 (y_p - y)$$

However, there is need for more efficacious methods of whitening the teeth.

## Summary of the Invention

We have determined that a composite comprising a specific plate shape particulate mineral such as kaolinite and a pigment having CIELAB hue angle "h" of 220 to 320 degrees, such as Blue Covarine, is not just suitable for use in oral care products like toothpastes, but its use leads to significant enhancement in Whiteness Index (WIO, Whiteness Index Observed) which is universally accepted means of measuring and reporting the efficacy of such compositions. The effect is seen in experiments conducted on bovine teeth. The invention permits reduction in the actual amount of the pigment that otherwise would be needed in oral care products like toothpastes for noticeable effect.

In accordance with a first aspect is disclosed a composite material comprising:

(i) a pigment having CIELAB hue angle "h" of 220 to 320 degrees; and,
(ii) a non-expanding bipolar 1:1 or 2:1:1 clay comprising alternating tetrahedral and octahedral sheets terminating with a tetrahedral sheet at one external surface plane and an octahedral sheet at another external surface plane;

Wherein the pigment is attached to coordinating cations on the external surface of the octahedral surface plane or the pigment is present primarily on the surface of the clay. In accordance with a second aspect is disclosed an oral care composition comprising a composite material of the first aspect.

In accordance with a third aspect is disclosed non-therapeutic use of a composite material of the first aspect of the invention for whitening teeth.

In accordance with a fourth aspect is disclosed a process of preparing a composite material of the first aspect comprising the steps of:

(i) preparing aqueous dispersion of a non-expanding bipolar 1:1 or 2:1:1 clay comprising alternating tetrahedral and octahedral sheets terminating with a tetrahedral sheet at one external surface plane and an octahedral sheet at another external surface plane of pH in the range of 4 to 10;
(ii) mixing said dispersion with a pigment having CIELAB hue angle "h" of 220 to 320 degrees; and,
(iii) heating said dispersion of step (ii) to 60 to 95 °C to evaporate said water.

Any feature of one aspect of the present invention may be utilized in any other aspect of the invention. The word "comprising" is intended to mean "including" but not necessarily "consisting of" or "composed of." In other words, the listed steps or options need not be exhaustive. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

All references to the terms wt% or % by weight shall be construed as referring to the % of the concerned ingredient by weight of the composition, unless indicated to the contrary.

## Detailed Description of the Invention

When referring to the pH of the composition, the pH is measured when 1 part by weight of the composition is uniformly dispersed and/or dissolved in 20 parts by weight pure water at 25 °C. In particular, the pH may be measured by manually mixing 1 g composition with 20 ml water for 30 seconds, then immediately testing it with indicator paper or a pH meter.

"Substantially free of, as used herein, means comprising less than 1.5%, and preferably less than 1.0%, and more preferably less than 0.75% and more preferably still less than 0.5%, and even more preferably less than 0.1 % and most preferably from 0.0 to 0.01 % by weight, based on total weight of the composition.

Viscosity of the composition is the value observed and recorded at room temperature (25 °C) with a Brookfield Viscometer, Spindle No. 4 and at a speed of 5 rpm. Values are quoted in centipoises (cP = mPa.s) unless otherwise specified.

Stained teeth could become troublesome, whether the cause for it is intrinsic or an extrinsic factor or agent like certain beverages and chlorhexidine. The accumulation of stains on teeth poses an aesthetic problem for some individuals. Stains are usually extrinsic in nature and generally represent discoloration of the pellicle or plaque. The exact mechanisms involved in stain formation may not be fully understood. It is suggested that pigments produced by chromagenic bacteria, colored products from the chemical transformation of pellicle components and adsorption/retention of dietary constituents contribute to extrinsic stains. The dietary factors which appear to contribute heavily towards staining include coffee, tea, and red wine, as well as smoking. In addition to chlorhexidine, staining is promoted by other cationic antimicrobial agents such as hexetidine or quaternary ammonium compounds.

The index proposed by Luo et al (known as WIO) has the same functional form as some of the previous whiteness indices but the parameters are different and are optimized specifically for the evaluation of whiteness in teeth. It is regarded as most appropriate for assessing changes in teeth whiteness.

The pigment

A pigment is generally understood as a material which is insoluble in the relevant medium under given conditions. This is in contrast to dyes such as triarylmethane dyes, which are soluble in the medium, which in most cases is water.

The term composite is used to differentiate the material from a mere admixture of the pigment and the clay. The bond or bonds that get formed between the two substances are not fully understood yet. It is preferred that in the composite material the pigment is attached to coordinating cations on the external surface of the octahedral surface plane. Alternatively, and more preferably, the pigment is present primarily on the surface of the clay. This can be visualized and confirmed by XRD studies, in which it is observed that there is no significant change in the "d" spacing which indicates that the pigment is primarily present on surface of the clay.

A detailed description of hue angles may be found on p57 of Colour Chemistry 3rd edition by H. Zollinger published by Wiley-VCH. While the preferred single pigment is blue or violet, the same effect may be achieved through mixing pigments outside of this h range; for example, such a hue angle may also be obtained by mixing a red and green-blue pigment to yield a blue or violet shaded pigment.

It is preferred that the pigment is a blue pigment. More preferably it is selected from alpha copper phthalocyanines which designated as C.I. Pigment Blue 15, more preferably Pigment Blue 15:1, 15:2, 15:3, 15:4, 15:5 or 15:6, most preferably 15:1. Particularly preferred is Blue Covarine. A commercially available example is Covarine Blue W 6795, ex Sensient Cosmetic Technologies/LCW. Mixtures of any of the above described materials may also be used.

Examples of suitable blue pigments include inorganic blue pigments such as iron blue (C.I. 77510) and ultramarine blue (C.I. 77007). A preferred class of blue pigments for use in the invention are organic blue pigments such as phthalocyanine blue pigments. Phthalocyanines are organometallic compounds containing four symmetrically arranged isoindole rings connected in a 16- membered ring linking with alternate carbon and nitrogen atoms. Most phthalocyanines contain a central, coordinated metal ion such as copper. Copper phthalocyanines have the basic structure:

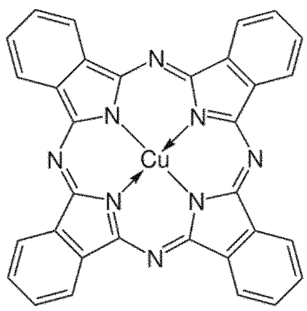

Phthalocyanine blue pigments exhibit more than one crystal modification, which differ in terms of coloristics. Methods have been developed to phase-stabilise the phthalocyanine pigment molecule in order to prevent conversion to a different crystal modification. An example is minor chemical modification of the molecule, for instance partial chlorination. Methods have also been developed to stabilise the phthalocyanine pigment molecule against flocculation during pigment application. An example is admixture of other agents to the molecule, such as surface active additives to the pigment molecule. The following pigments are illustrative phthalocyanine blue pigments preferably comprised in the composite material according to the invention:

| C.I. Generic Name | C.I. Constitution Number | Crystal modification; Type | Number of halogen atoms |
|---|---|---|---|
| Pigment Blue 15 | 74 160 | alpha | - |

(continued)

| C.I. Generic Name | C.I. Constitution Number | Crystal modification; Type | Number of halogen atoms |
|---|---|---|---|
| Pigment Blue 15: 1 | 74 160 | alpha; phase stabilised | 0.5-1 Cl |
| Pigment Blue 15: 2 | 74 160 | alpha; phase & flocculation stabilised | 0.5-1 Cl |
| Pigment Blue 15: 3 | 74 160 | beta | - |
| Pigment Blue 15: 4 | 74 160 | beta; flocculation stabilised | - |
| Pigment Blue 15: 6 | 74 160 | epsilon | - |
| Pigment Blue 16 | 74 100 | gamma; metal-free | - |

The clay

It is preferred that when the clay is of the 1:1 type, it belongs to kaolinite or serpentine subgroup. 1:1 clays preferred according to the present invention include kaolinite and serpentine subgroups of minerals. The species included within kaolinite subgroup are particularly preferred viz. kaolinite, dickite, halloysite and nacrite. The species included within serpentine subgroup are chrysolite, lizardite, and amesite.

2:1:1 clays preferred according to the present invention include chlorite group of minerals. Chlorite is also erroneously referred to as a 2:2 clay by some mineralogists. The chlorite comprises tetrahedral-octahedral-tetrahedral sheets like 2:1 clays, with extra weakly bound brucite like layer between tetrahedral layers.

The tetrahedral sheet preferably comprises coordinating tetrahedral cation of silicon. The tetrahedral sheet may also comprise isomorphously substituted coordinating tetrahedral cations which are not silicon. Isomorphously substituted coordinating tetrahedral cations include, but are not limited to, cations of aluminium, iron or boron.

The octahedral sheet preferably comprises coordinating octahedral cation of aluminium. The octahedral sheet may also comprise isomorphously substituted coordinating octahedral cations which are not aluminium. Isomorphously substituted coordinating octahedral cations include cations of magnesium or iron.

It is preferred that the amount of pigment in said composite material is 0.05 to 10 % by weight of the material. Therefore, when a particular amount of the pigment is intended to be included in an oral care composition, the formulation scientist needs to dose an amount of the composite equivalent to (or which contains) the desired amount of the pigment.

It is preferred that the particle size of the clay in the composite is from 0.1 to 10 $\mu$m.

It is particularly preferred that the composite material of the invention comprises Blue Covarine as the pigment and a non-expanding bipolar 1:1 clay, which more preferably is kaolinite. It is particularly preferred that the amount of pigment in the composite particle of the invention is 0.1 to 1 wt% of the particle.

Process

In accordance with another aspect is disclosed a process of preparing a composite material of the first aspect, comprising the steps of:

(i) preparing aqueous dispersion of a non-expanding bipolar 1:1 or 2:1:1 clay comprising alternating tetrahedral and octahedral sheets terminating with a tetrahedral sheet at one external surface plane and an octahedral sheet at another external surface plane of pH in the range of 4 to 10;
(ii) mixing said dispersion with a pigment having CIELAB hue angle "h" of 220 to 320 degrees; and,
(iii) heating said dispersion of step (ii) to 60 to 95 °C to evaporate said water.

For example, a preferred aspect, 250 mg Blue Covarine is added to 1 litre water in a container. The mix is stirred for 30 minutes. Thereafter, 100 g kaolinite is added under stirring. The contents is heated to 95 °C under stirring at which stage most of the water evaporates from the dispersion. The composite material is further dried at 45 °C for 24 hours.

Oral care composition of the invention

In accordance with another aspect is disclosed an oral care composition comprising a composite material of the first aspect. Preferably the oral care composition is a toothpaste, a toothpowder or a mouthwash. Other known forms include chewing gums and lozenges, strips and gels.

It is preferred that the oral care compositions of the invention comprise from 0.5 to 5 wt% of the composite particle, more preferably 1 to 3 wt% and most preferably 1.5 to 3 wt% of the oral care composition.

It is preferred that when the oral care composition according to the invention is a toothpaste, the toothpaste comprises at least one of calcium-based abrasive or silica-based abrasive.

Toothpastes are also known as dentifrices. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically, the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

An oral care composition according to the invention will usually contain a liquid continuous phase in an amount of from 40 to 99% by weight based on the total weight of the dentifrice. Such a liquid continuous phase will typically comprise a mixture of water and polyhydric alcohol in various relative amounts, with the amount of water generally ranging from 10 to 45% by weight (based on the total weight of the dentifrice) and the amount of polyhydric alcohol generally ranging from 30 to 70% by weight (based on the total weight of the dentifrice). Typical polyhydric alcohols include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof.

An oral care composition according to the invention will generally contain further ingredients to enhance performance and/or consumer acceptability such as abrasive cleaning agent, binder or thickening agent, and surfactant.

For example, a dentifrice will usually comprise an abrasive cleaning agent in an amount of from 3 to 75% by weight based on the total weight of the dentifrice. Suitable abrasive cleaning agents include silica xerogels, hydrogels and aerogels and precipitated particulate silicas; calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof. Examples of abrasives include abrasive amorphous silica particles which have a weight mean particle size (d50) ranging from 3 to 15 microns. Preferred abrasive amorphous silica particles for use in the composition of the invention have a weight mean particle size in the range 3 to 6 microns. Preferably, the abrasive amorphous silica particles employed are precipitated silica. Suitable precipitated silicas for use as abrasive amorphous silica particles in the invention are commercially available and include those marketed by PQ Corporation under the trade names SORBOSIL® AC 43, AC77, AC35 and SORBOSIL® AC 33. Mixtures of any of the above described materials may also be used. The level of abrasive amorphous silica particles (as defined above) generally ranges from 0.05 to 5%, preferably from 0.1 to 3%, more preferably from 0.2 to 0.8%, by total weight abrasive amorphous silica particles (as defined above) based on the total weight of the composition.

Furthermore, the dentifrice will usually contain a binder or thickening agent in an amount of from 0.5 to 10% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth), finely divided silicas, hectorites, colloidal magnesium aluminium silicates and mixtures thereof.

Furthermore, the dentifrice will usually contain a surfactant in an amount of from 0.2 to 5% by weight based on the total weight of the dentifrice. Suitable surfactants include anionic surfactants, such as the sodium, magnesium, ammonium or ethanolamine salts of C8 to C18 alkyl sulphates (for example sodium lauryl sulphate), C8 to C18 alkyl sulphosuccinates (for example dioctyl sodium sulphosuccinate), C8 to C18 alkyl sulphoacetates (such as sodium lauryl sulphoacetate), C8 to C18 alkyl sarcosinates (such as sodium lauryl sarcosinate), C8 to C18 alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used.

Oral care compositions of the invention further preferably comprise a zinc salt, preferably zinc sulphate or zinc chloride, more preferably zinc sulphate heptahydrate. Preferably the level of zinc salt is from 0.05 to 1.0 wt percent of the total composition more preferably from 0.1 to 0.5wt percent. It is preferable if the level of ethanol in the total composition is less than 0.1 wt percent. Compositions of the invention may comprise a preservative, a preferred preservative is

sodium benzoate. Preferably the level of preservative is from 0.1 to 1 wt percent of the total composition. It is preferred that pH of the composition at 20 °C is from 4 to 10, more preferably 7 to 9.

The composition of the invention may also comprise a deposition aid. The term "deposition aid" in the context of this invention generally means a material which further aids deposition of whitening agents from the composition. An example is polystyrene sulphonates. Another examples is Gantrez® polymers.

Another preferred type of product form is a mouthwash. The term "mouthwash" generally denotes liquid formulations which are used to rinse the surfaces of the oral cavity and provide the user with a sensation of oral cleanliness and refreshment. The mouthwash is an oral composition that is not intentionally swallowed for purposes of systemic administration of therapeutic agents, but is applied to the oral cavity, used to treat the oral cavity and then expectorated.

A mouthwash composition will usually contain an aqueous continuous phase. The amount of water generally ranges from 70 to 99% by weight based on the total weight of the mouthwash.

A mouthwash composition according to the invention will generally contain further ingredients to enhance performance and/or consumer acceptability, such as the humectants and surfactants mentioned above for dentifrices. The amount of humectant generally ranges from 5 to 20% by weight based on the total weight of the mouthwash and the amount of surfactant generally ranges from 0.1 to 5% by weight based on the total weight of the mouthwash.

Compositions of the present invention (such as in particular dentifrices or mouthwashes) may also contain further optional ingredients customary in the art such as fluoride ion sources, anticalculus agents, buffers, flavouring agents, sweetening agents, colouring agents, opacifying agents, preservatives, antisensitivity agents and antimicrobial agents.

Use of the composition in the context of this invention typically involves application of the composition to the oral cavity, for a recommended time, before being expectorated. The preferred time application time being from 10 to 60 seconds.

The method according to the Invention

In accordance with another aspect is disclosed a non-therapeutic method of whitening teeth comprising a step of applying thereto a composite material of the first aspect, more preferably in the form of an oral care composition, particularly a toothpaste.

The method of whitening the teeth may be performed in a dentist's office or clinic under ordinary or supervised conditions. Alternatively, and more preferably the method is performed by an informed user at home or in other words, without any medical supervision when the method is non-therapeutic.

The composition and method of the invention is useful to whiten teeth. This means that the composition is useful to preserve the white appearance of teeth or to restore the appearance periodically. Alternatively, the composition and the method is useful to lighten the teeth which are discoloured due to extrinsic or intrinsic stains (e.g., tobacco, coffee, tea and/or food or wine stains), fluorosis, developmental disturbances, bacteria, genetics, tetracycline antibiotics, trauma, blood decomposition, pigments present during development of teeth.

In one aspect the method is non-therapeutic. Preferably such a method is a cosmetic method. A therapeutic treatment starts from a pathological state, whereas a non-therapeutic treatment starts from a normal, healthy state. Application of such a method to a person is to improve his bodily appearance (cosmetic treatment).

In another aspect the method is therapeutic in nature. Treatment by therapy is defined as any treatment which is designed to cure, alleviate, remove or lessen the symptoms of, or prevent or reduce the possibility of contracting any disorder or malfunction of the human body, in this case it is teeth.

Use in accordance with the Invention

In accordance with yet another aspect is disclosed non-therapeutic use of a composite material in accordance with the invention for whitening teeth.

. The part of the description related to the method of the invention is also applicable *mutatis mutandis* to this aspect of the invention.

The following non-limiting examples will more fully illustrate the embodiments of this invention. All parts, percentages and proportions referred to herein and in the appended claims are by weight unless otherwise illustrated.

## Examples

Example 1: Experiments on Bovine blocks

A convenient measure of the whiteness of teeth is delta b* which is measured using a Chromameter. Negative values of delta b* indicate a colour shift from yellow to blue which has been shown to be one of the primary drivers of

whiteness as perceived by consumer.

A composite according to the invention was prepared by the procedure disclosed on page 9.:
The composite material comprised 0.1 wt% Blue Covarine and balance of clay.

The above material was then used in a formulation of an oral composition (toothpaste - gel). For the purpose of comparison, two other compositions were also prepared. Details are shown in Table 1. The composition bearing reference code 1 was in accordance with the invention, the others were not.

**Table 1**

| **Ingredient/wt%** | **Composition Reference Code** | | | |
|---|---|---|---|---|
| | **A** | **B** | **C** | **1** |
| Sorbitol (70% active) | 65.0 | 65.0 | 65.0 | 65.0 |
| Polyethylene glycol | 1.5 | 1.5 | 1.5 | 1.5 |
| Gantrez® | 1.0 | 1.0 | 1.0 | 1.0 |
| Abrasive silica | 14.0 | 14.0 | 14.0 | 14.0 |
| Thickening silica | 1.5 | 1.5 | 1.5 | 1.5 |
| Sodium lauryl sulphate | 1.8 | 1.8 | 1.8 | 1.8 |
| Kaolinite | 2.0 | 0 | 2.0 | 0 |
| Blue Covarine | 0 | 0.002 | 0.002 | 0 |
| Composite material of the invention | 0 | 0 | 0 | 2.0 |
| Water and other minors | To 100 | To 100 | To 100 | To 100 |

Seven bovine blocks were prepared for each group and were kept in deionized water for complete hydration. The baseline L*, a*, b* of the bovine blocks was measured by KONICA MINOLTA® CM2600D Spectrophotometer. Aqueous slurries of the toothpastes were prepared by mixing the concerned paste with deionized water (w/w=1:2) such that final concentration of Blue Covarine in each case was at 0.025% w/w. Thereafter the bovine blocks were brushed for one minute with the concerned slurry on a WIRA® brushing machine maintaining the load at 175 g. Thereafter the blocks were rinsed twice each time with 50 ml water for 20 steps. After this step the L*a*b* values were recorded based on which the change in WIO was calculated for each case.

The observations are summarised in Table 2

**Table 2**

| **Composition Reference code** | **Mean Delta WIO** | **Standard Deviation** |
|---|---|---|
| A | 2.057 | 0.318 |
| B | 2.843 | 0.021 |
| C | 6.076 | 0.248 |
| 1 | 7.203 | 0.189 |

The data clearly indicate that the Composition 1 was significantly more than the index observed in the case of all the other compositions and the difference between the observed values of C v/s 1 was more than 1 unit which reflects a substantially significant improvement in efficacy of whitening the teeth despite the fact that Composition C had an equivalent amount of the pigment and the same clay.

## Claims

**1.** A composite material comprising:

(i) 0.05 to 10% by weight of the material of a pigment having CIELAB hue angle "h" of 220 to 320 degrees; and,
(ii) a non-expanding bipolar 1:1 or 2:1:1 clay comprising alternating tetrahedral and octahedral sheets terminating

with a tetrahedral sheet at one external surface plane and an octahedral sheet at another external surface plane;

Wherein the pigment is attached to coordinating cations on the external surface of the octahedral surface plane or the pigment is present primarily on the surface of the clay.

**2.** A composite material as claimed in claim 1 wherein said pigment is a blue pigment.

**3.** A composite material as claimed in claim 2 wherein said blue pigment is a phthalocyanine blue pigment.

**4.** A composite as claimed in claim 3 wherein said phthalocyanine blue pigment is selected from alpha copper phthalocyanines designated as C.I. Pigment Blue 15, C.I. Pigment Blue 15:1, or C.I. Pigment Blue 15:2 or 15:3, 15:4, 15:5 or 15:6.

**5.** A composite material as claimed in any of claims 1 to 4 wherein said pigment is present primarily on the surface of said clay.

**6.** A composite material as claimed in any of claims 1 to 5 wherein when said clay is of the 1:1 type, it belongs to kaolinite or serpentine subgroup.

**7.** A composite as claimed in claim 6 wherein said clay is kaolinite, dickite, halloysite, nacrite, chrysolite, lizardite or amesite.

**8.** A composite material as claimed in any of claims 1 to 7 wherein particle size of the clay in said composite is from 0.1 to 10 μm.

**9.** An oral care composition comprising a composite material as claimed in any of claims 1 to 8.

**10.** An oral care composition as claimed in claim 9 wherein said composition is a toothpaste, a toothpowder or a mouthwash.

**11.** Non-therapeutic use of a composite material as claimed in any of claims 1 to 8 for whitening the teeth.

**12.** A non-therapeutic method of whitening teeth comprising a step of applying thereto a composite material as claimed in any of claims 1 to 8.

**13.** A process of preparing a composite material as claimed in claim 1 comprising the steps of:

(i) preparing aqueous dispersion of a non-expanding bipolar 1:1 or 2:1:1 clay comprising alternating tetrahedral and octahedral sheets terminating with a tetrahedral sheet at one external surface plane and an octahedral sheet at another external surface plane of pH in the range of 4 to 10;
(ii) mixing said dispersion with a pigment having CIELAB hue angle "h" of 220 to 320 degrees; and,
(iii) heating said dispersion of step (ii) to 60 to 95 °C to evaporate said water.

## Patentansprüche

**1.** Verbundmaterial, umfassend:

(i) 0,05 bis 10 Gewichts-% des Materials eines Pigments mit einem CIELAB-Farbtonwinkel "h" von 220 bis 320 Grad und
(ii) einen nicht-expandieren bipolaren 1:1- oder 2:1:1-Ton, umfassend abwechselnde tetraedrische und oktaedrische Schichten, die mit einer tetraedrischen Schicht an einer äußeren Oberflächenebene und einer oktaedrischen Schicht an einer anderen äußeren Oberflächenebene enden,

wobei das Pigment mit koordinierenden Kationen auf der äußeren Oberfläche der oktaedrischen Oberflächenebene verbunden ist oder das Pigment hauptsächlich an der Oberfläche des Tons vorliegt.

**2.** Verbundmaterial, wie im Anspruch 1 beansprucht, wobei das Pigment ein Blaupigment ist.

3. Verbundmaterial, wie im Anspruch 2 beansprucht, wobei das Blaupigmnet ein Phthalocyanin-Blaupigment ist.

4. Verbundmaterial, wie im Anspruch 3 beansprucht, wobei das Phthalocyanin-Blaupigment aus Alpha-Kupferphthalocyaninen ausgewählt ist, bezeichnet als C.I. Pigment Blue 15, C.I. Pigment Blue 15:1 oder C.I. Pigment Blue 15:2 oder 15:3, 15:4, 15:5 oder 15:6.

5. Verbundmaterial, wie in irgendeinem der Ansprüche 1 bis 4 beansprucht, wobei das Pigment hauptsächlich auf der Oberfläche des Tons vorliegt.

6. Verbundmaterial, wie in irgendeinem der Ansprüche 1 bis 5 beansprucht, wobei, wenn der Ton vom 1:1-Typ ist, er zur Kaolinit- oder Serpentin-Untergruppe gehört.

7. Verbundmaterial, wie im Anspruch 6 beansprucht, wobei der Ton Kaolinit, Dickit, Halloysit, Nacrit, Chrysolith, Lizardit, oder Amesit ist.

8. Verbundmaterial, wie in irgendeinem der Ansprüche 1 bis 7 beansprucht, wobei die Teilchengröße des Tons in dem Verbundmaterial 0,1 bis 10 $\mu$m beträgt.

9. Mundpflegezusammensetzung, umfassend ein Verbundmaterial, wie in irgendeinem der Ansprüche 1 bis 8 beansprucht.

10. Mundpflegezusammensetzung, wie im Anspruch 9 beansprucht, wobei die Zusammensetzung eine Zahnpasta, ein Zahnpulver oder ein Mundwasser ist.

11. Nicht-therapeutische Verwendung eines Verbundmaterials, wie in irgendeinem der Ansprüche 1 bis 8 beansprucht, zum Aufhellen von Zähnen.

12. Nicht-therapeutisches Verfahren zum Aufhellen von Zähnen, umfassend einen Schritt des darauf Auftragens eines Verbundmaterials, wie in irgendeinem der Ansprüche 1 bis 8 beansprucht.

13. Verfahren zur Herstellung eines Verbundmaterials, wie im Anspruch 1 beansprucht, umfassend die Schritte:

(i) Herstellen einer wässrigen Dispersion eines nicht-expandierenden bipolaren 1:1- oder 2:1:1-Tons, umfassend abwechselnde tetraedrische und oktaedrische Schichten, die mit einer tetraedrischen Schicht an einer äußeren Oberflächenebene und einer oktaedrischen Schicht an einer anderen äußeren Oberflächenebene enden, mit einem pH in dem Bereich von 4 bis 10;
(ii) Mischen der Dispersion mit einem Pigment eines CIELAB-Farbtonwinkels "h" von 220 bis 320 Grad und
(iii) Erhitzen der Dispersion von Schritt (ii) auf 60 bis 95°C, um das Wasser zu verdampfen.

**Revendications**

1. Matériau composite comprenant :

(i) de 0,05 à 10 % en masse du matériau d'un pigment ayant un angle de teinte CIELAB "h" de 220 à 320 degrés ; et,
(ii) une argile bipolaire 1 : 1 ou 2 : 1 : 1 non-expansée comprenant des feuilles alternées tétraédriques et octaédriques se terminant avec une feuille tétraédrique sur un plan de surface externe et une feuille octaédrique sur un autre plan de surface externe ;

où le pigment est attaché à des cations de coordination sur la surface externe du plan de surface octaédrique ou le pigment est présent principalement sur la surface de l'argile.

2. Matériau composite selon la revendication 1, où ledit pigment est un pigment bleu.

3. Matériau composite selon la revendication 2, où ledit pigment bleu est un pigment bleu de phtalocyanine.

4. Composite selon la revendication 3, où ledit pigment bleu de phtalocyanine est choisi parmi des phtalocyanines de

cuivre alpha désignées par C.I. Pigment Blue 15, C.I. Pigment Blue 15 : 1, ou C.I. Pigment Blue 15 : 2 ou 15 : 3, 15 : 4, 15 : 5 ou 15 : 6.

**5.** Matériau composite selon l'une quelconque des revendications 1 à 4, où ledit pigment est présent principalement sur la surface de ladite argile.

**6.** Matériau composite selon l'une quelconque des revendications 1 à 5, où ladite argile est du type 1 : 1, elle appartient au sous-groupe de kaolinite ou serpentine.

**7.** Composite selon la revendication 6, où ladite argile est la kaolinite, dickite, halloysite, nacrite, chrysolite, lizardite ou amésite.

**8.** Matériau composite selon l'une quelconque des revendications 1 à 7, où la taille de particule de l'argile dans ledit composite est de 0,1 à 10 $\mu$m.

**9.** Composition pour le soin oral comprenant un matériau composite selon l'une quelconque des revendications 1 à 8.

**10.** Composition pour le soin oral selon la revendication 9, où ladite composition est un dentifrice, une poudre dentaire ou une eau de bouche.

**11.** Utilisation non-thérapeutique d'un matériau composite selon l'une quelconque des revendications 1 à 8 pour blanchir les dents.

**12.** Procédé non-thérapeutique de blanchiment des dents comprenant une étape d'application à celles-ci d'un matériau composite selon l'une quelconque des revendications 1 à 8.

**13.** Procédé de préparation d'un matériau composite selon la revendication 1 comprenant les étapes de :

(i) préparation d'une dispersion aqueuse d'une argile bipolaire 1 : 1 ou 2 : 1 :1 non-expansée comprenant des feuilles tétraédriques et octaédriques alternées se terminant avec une feuille tétraédrique sur un plan de surface externe et une feuille octaédrique sur un autre plan de surface externe de pH dans l'intervalle de 4 à 10 ;
(ii) mélange de ladite dispersion avec un pigment ayant un angle de teinte CIELAB "h" de 220 à 320 degrés ; et,
(iii) chauffage de ladite dispersion de l'étape (ii) à de 60 à 95°C pour faire évaporer ladite eau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 12123241 A1
- EP 1935395 B1
- US 2013129642 A
- US 20160331653 A1
- US 20161296434 A1
- WO 2012123241 A2
- WO 11036029 A1

### Non-patent literature cited in the description

- **GARCIA JA ; RUBINO M ; ROMERO J ; HITA E.** *Measuring The Whiteness Of Human Teeth. Color Research and Application,* 1993, vol. 18, 349-52
- **H. ZOLLINGER.** Colour Chemistry. Wiley-VCH